# EUROPEAN PATENT SPECIFICATION

(11) **EP 1 251 850 B1**
(45) Date of publication and mention of the grant of the patent: **21.06.2006**
(21) Application number: 01908685.9
(22) Date of filing: 24.01.2001
(51) Int. Cl.: A61K 31/4188, A61P 35/00

(54) **USE OF A COMBINATION PREPARATION IN CANCER THERAPY**
KOMBINATIONSPRÄPARAT ZUR KREBSTHERAPIE
PREPARATION COMBINEE DANS LA THERAPIE DU CANCER

(30) Priority: 26.01.2000 US 178541 P
(43) Date of publication of application: 30.10.2002
(73) Proprietor: SCHERING CORPORATION, Kenilworth, New Jersey 07033-0530 (US)
(72) Inventor: HOUGHTON, Peter, J., Memphis, TN 38103 (US)
(74) Representative: UEXKÜLL & STOLBERG
(86) International application number: PCT/US2001/002372
(87) International publication number: WO 2001/054678

(56) References cited:
- WO-A-97/07804
- KANO Y ET AL: "EFFECTS OF CPT-11 IN COMBINATION WITH OTHER ANTI-CANCER AGENTS IN CULTURE" INTERNATIONAL JOURNAL OF CANCER,US,NEW YORK, NY, vol. 50, no. 4, 20 February 1992 (1992-02-20), pages 604-610, XP000563779 ISSN: 0020-7136
- EDER JP ET AL: "Sequence effect of irinotecan (CPT-11) and topoisomerase II inhibitors in vivo" CANCER CHEMOTHERAPY AND PHARMACOLOGY,DE,SPRINGER VERLAG, BERLIN, vol. 42, no. 4, 1998, pages 327-335, XP002112007 ISSN: 0344-5704
- PLOWMAN J ET AL: "PRECLINICAL ANTITUMOR ACTIVITY OF TEMOZOLOMIDE IN MICE: EFFICACY AGAINST HUMAN BRAIN TUMOR XENOGRAFTS AND SYNERGISM WITH 1,3-BIS(2-CHLOROETHYL)-1-NITROSOUREA" CANCER RESEARCH,US,AMERICAN ASSOCIATION FOR CANCER RESEARCH, BALTIMORE, MD, vol. 54, no. 14, 1994, pages 3793-3799, XP000914803 ISSN: 0008-5472
- NEWLANDS E S ET AL: "TEMOZOLOMIDE: A REVIEW OF ITS DISCOVERY, CHEMICAL PROPERTIES, PRE-CLINICAL DEVELOPMENT AND CLINICAL TRIALS" CANCER TREATMENT REVIEWS,US,SAUNDERS, vol. 23, no. 1, 1997, pages 35-61, XP000921344 ISSN: 0305-7372
- DATABASE CANCERLIT [Online] AN: 97600169

## Description

### BACKGROUND OF THE INVENTION

Despite the numerous advances in cancer treatment, the well-known life style changes that can greatly reduce the risk of cancer, and the early warning signs that some cancers provide, many patients still develop cancer for which no conventional therapies are available that offer any reasonable hope of cure or significant pallation.

Temozolomide is known for its anti-tumor effects. For example, in one study clinical responses were achieved in 17% of patients having advanced melanoma (Newlands ES, et al., Br. J Cancer 65 (2) 287-291, 1992). In another study, a clinical response was achieved in 21 % of patients with advanced melanoma (Journal of Clinical Oncology, Vol 13, No. 4 (April), 1995, pp. 910-913). However, temozolomide is not always effective and has dose-limiting side effects, such as hematologic toxicity, myelosuppression, anemia, leukopenia, etc.

Irinotecan, also known as CPT-11, is also known to have anti-cancer effects. See, for example, U.S. Patent. Nos. 5,955,466 and 4,604,463. However, this treatment is not always effective and sometimes results in intolerable side effects related to the dosage and duration of therapy, such as diarrhea.

Combinations of temozolomide with the topoisomerase I inhibitor topotecan have been shown to possess supra-addidive activity (Cancer Treat. Rev 1997, 23, 35-61 and Proc. Annu. Meet. Am. Assoc. Cancer Res., 1996, 37, A 1988)

There is a need for a method for treating cancers with higher response rates or reduced side effects, or both.

### SUMMARY OF THE INVENTION

The present invention provides the use of a therapeutically effective amount of temozolomide for the preparation of a pharmaceutical composition for treating a human patient afflicted with cancer, wherein the treatment comprises administering temozolomide to such a patient for a time period sufficient to effect at least a partial tumor response.

The invention also provides the use of a therapeutically effective amount of irinotecan for the preparation of a pharmaceutical composition for treating a human patient afflicted with cancer, wherein the treatment comprises administering temozolomide and irinotecan to such a patient for a time period sufficient to effect at least a partial tumor response.

The temozolomide is administered to the patient in combination with the irinotecan; that is, the temozolomide and irinotecan doses are administered during the same treatment cycle. Preferred dosing schedules are described below.

In a further aspect of the present invention, a medical kit for treating a cancer patient is provided, comprising:
(a) a supply of temozolomide;
(b) a supply of irinotecan; and
(c) printed instructions for administering temozolomide and irinotecan to a cancer patient.

### DETAILED DESCRIPTION

The term "temozolomide" is intended to mean a compound having the formula: 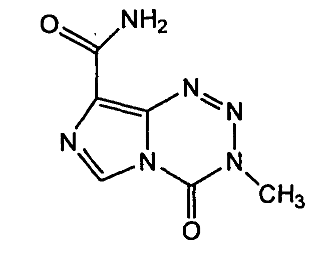 One chemical name for temozolomide is 3,4-dihydro-3-methyl-4-oxoimidazo-[5,1-d]1,2,3,4-tetrazin-8-carboximide. The synthesis of temozolomide is well known, see for example, Stevens et al., J. Med. Chem., 1984, 27, 196-201 and Wang et al., J. Chem. Soc., Chem. Commun., 1994, pp. 1687-1688.

The term "irinotecan" or "CPT-11" is intended to mean a compound having the formula: 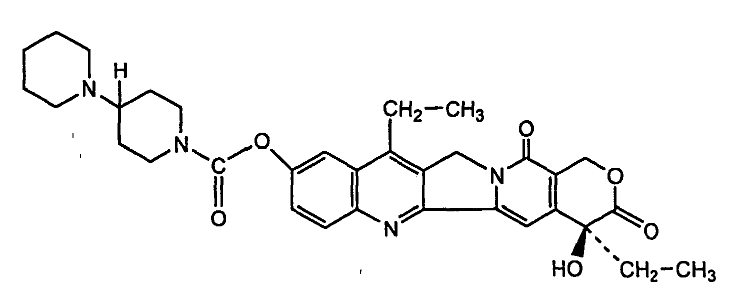 or a pharmaceutically acceptable salt thereof. Irinotecan is preferably in the form of a hydrochloride salt. The synthesis of irinotecan and its salts is well known, and is described, for example, in U.S. Patent 4,604,463. Irinotecan hydrochloride is available commercially in the form of CAMPTOSAR Injection, sold by Pharmacia & Upjohn.

As used herein, the term "mg/m²" refers to a dose measured in milligrams per square meter of body surface of the patient.

As used herein, the term "mg/m²/day" refers to a daily dose measured in milligrams per square meter of body surface area of the patient.
Examples of cancers treatable by this invention include, but are not limited to melanoma; high grade glioma, glioblastoma and other brain cancers; lung cancer; breast cancer; testicular cancer; gastro-intestinal cancers including colon, rectal, pancreatic, and gastric cancers, hepatocellular carcinoma; head and neck cancers; prostate cancer, renal cell carcinoma, adenocarcinoma; sarcomas; lymphomas, leukemias; and mycosis fungoides. This invention contemplates treating these cancers and other cancers at any stage from the discovery of the cancer to the advanced stage. The invention includes treatment of the primary cancer and metastases thereof.

A person suffering from advanced cancer may exhibit one or more of the following signs or symptoms:
(a) presence of cancerous tumor,
(b) fatigue,
(c) pain,
(d) decreased performance status from tumor burden, and
(e) the well known symptoms associated with each specific cancer.
To practice the invention, temozolomide and irinotecan are used for the preparation of a pharmaceutical composition for administration to the patient exhibiting one or more of the above signs or symptoms in amounts sufficient to eliminate or at least alleviate one or more of the signs or symptoms.

Preferably, the temozolomide and irinotecan are administered over repeated 21 or 28 day cycles. Preferably, the total amount of irinotecan administered over a 21 or 28 day period, ranges from 3 to 400 mg per m² of the patient's body surface. Preferably, temozolomide is administered over a 5-21 day period in a dose from 50 to 200 mg/m²/day.

In one preferred 21 day cycle, the irinotecan is administered for 5 consecutive days at a daily dose of from 10 to 40 mg /m² during the first week, and for 5 consecutive days at a daily dose of from 10 to 40 mg /m² during the second week, followed by a third week in which irinotecan is not administered. During this 21 day cycle, the temozolomide may be administered for 5 consecutive days at a daily dose of from 100 to 200 mg /m² during the first week, followed by the second and third weeks in which temozolomide is not administered. Alternatively, the temozolomide may be administered for 5-7 consecutive days at a daily dose of from 100 to 200 mg/m² during the first and third weeks, or the first and second weeks.

In a variation of the 21 day cycle described above, the irinotecan is administered on a single day of the 21 day cycle in an amount of from 250 to 650 mg/m². In another variation of the 21 day cycle described above, the irinotecan is administered once a week during the 21 day cycle at a dose of from 100 to 125 mg /m². The cycles may be repeated for as long as necessary.

Preferably, temozolomide and irinotecan are both administered on the first day of the 21 day treatment cycle. It is also preferred that temozolomide be administered prior to administering irinotecan.

Preferably, Temozolomide was administered orally for the first 5 days of a 21 day cycle at doses ranging from 50 to 200 mg/m²/day. During this 21 day cycle, irinotecan was administered intravenously daily for the same 5-day period, and for an additional 5 day period during the second week at doses ranging from 10 to 40 mg/m²/day, followed by a third week where no drugs were administered. Three 21 day cycles were given.

In another embodiment of the invention, the temozolomide and irinotecan are administered over repeated 28 day cycles. In one preferred 28 day cycle, the irinotecan is administered once at a dose of 100 to 350 mg/m²/day. In an even more preferred 28 day cycle, the irinotecan is administered on one day, between day 1 through day 8 of said 28 day cycle, at a dose of 100 to 350 mg/m²/day. During this 28 day cycle, the temozolomide is administered on days 1-14 of said cycle, at a daily dose of 75 to 150 mg/m²/day.

Temozolomide may be administered orally in capsule form wherein it is admixed with conventional pharmaceutical carriers. Preferred temozolomide capsule formulations are as described below in Table 1:

**Table 1**

| **Ingredient** | **mg/Capsule** | | | |
|---|---|---|---|---|
| Temozolomide | 5 | 20 | 100 | 250 |
| Anhydrous Lactose NF | 132.8 | 182.2 | 175.7 | 154.3 |
| Sodium Starch Glycolate NF | 7.5 | 11.0 | 15.0 | 22.5 |
| Colloidal Silicon Diozide NF | 0.2 | 0.2 | 0.3 | 0.7 |
| Tartaric Acid NF | 1.5 | 2.2 | 3.0 | 9.0 |
| Steric Acid NF | 3.0 | 4.4 | 6.0 | 13.5 |
| Capsule Size* | 3 | 2 | 1 | 0 |

| | | | | |
|---|---|---|---|---|
| *White opaque, preservative-free, two-piece hard gelatin capsules | | | | |

It is preferred that the patient fast from all food or drink, except water, for one hour before temozolomide administration and for two hours after.

The irinotecan is preferably administered in either normal saline or 5% dextrose (USP) by intravenous infusion over a 60-90 minute period.

The treatment may be continued until a clinical response is achieved or until intolerable side effects are encountered. The dosages of temozolomide and/or irinotecan may be increased with each new treatment cycle, provided intolerable side effects are not encountered. The dosages may also be decreased, if intolerable side effects are encountered.

A common, but tolerable, side effect of temozolomide is nausea and vomiting. This can be alleviated by administering an anti-emetic in conjunction with the temozolomide. It is preferred that an anti-emetic be given p.o. about 30 minutes before temozolomide administration. Examples of anti-emetics that may be administered, include, but are not limited to Haldol, Benadryl, Ativan, Compazine, and Ondansetron.

Of course, other forms of administration of both active ingredients, as they become available, are contemplated, such as by nasal spray, transdermally, by suppository, by sustained release dosage form, by IV injection, etc. Any form of administration will work so long as the proper dosages are delivered without destroying the active ingredient.

The effectiveness of treatment may be assessed by conventional means, e.g., by determining whether the number and/or size of tumors has decreased. Elimination or alleviation of other known signs or symptoms of cancer, especially those listed previously, can also be used to evaluate the effectiveness of this invention.

The medical kit in accordance with the present invention may be in any form suitable for providing a supply of temozolomide and irinotecan, together with instructions for administering the two drugs. Examples include, but are not limited to, various containers (e.g., bottles, cartons, blister packs, and ampules) either accompanied by a package insert describing the dosing instructions, or wherein the dosing instructions are printed on, or affixed to the container.

### EXAMPLES

The antitumor activity of temozolomide alone, irinotecan alone, and the combination of temozolomide and irinotecan were evaluated against a series of human xenograft cancer models in immune-deprived mice. These models included neuroblastoma, glioblastoma and rhabdomyosarcoma. Mice with advanced subcutaneous tumors were treated according to the following schedule:
A. Temozolomide Alone: Temozolomide was administered orally for 5 consecutive days every 21 days, at doses ranging from 63 to 200 mg/m²/day, for three 21 day cycles.
B. Irinotecan Alone: Irinotecan was administered intravenously for 5 consecutive days during the first week, for 5 consecutive days during the second week, followed by a week in which irinotecan was not administered. Doses ranged from 0.46 to 8.3 mg/m²lday. Three 21 day cycles of treatment were given.
C. Combination of Temozolomide and Irinotecan: Temozolomide was administered orally for the first 5 days of a 21 day cycle at doses ranging from 63 to 200 mg/m²/day. Irinotecan was administered intravenously daily for the same 5-day period, and for an additional 5 day period during the second week at doses ranging from 0.46 to 8.3 mg/m²/day. No drugs were administered during the third week. Three 21 day cycles were given.

Tumor dimensions were determined every seven days, and tumor volumes calculated for up to 12 weeks after start of treatment. The combination of temozolomide with irinotecan demonstrated greater than additive activity, i.e., synergy, in five tumor models (three neuroblastoma, one rhabdomyosarcoma, and one glioblastoma model) and was at least additive in activity against a further two tumor models (one rhabdomyosarcoma, and one neuroblastoma model).

### D. Combination of Temozolomide and Irinotecan in vivo

Abbreviations as herein defined for this section, Example D: CR, complete response; PR, partial response; MCR, maintained CR at week 12; MMR mismatch repair; MGMT, O⁶-methylguanine-DNA methyltransferase; CPT-11, irinotecan [7-ethyl-10-(4-[1-piperidino)-1-piperidino]-carbonyloxy-camptothecin].

Note, for the dosages used in the below example, 200 mg/m² is equivalent to 66 mg/kg.

It has been have shown that the antitumor activity of camptothecins is highly schedule dependent. For example, the same total dose of CPT-11 given over 10 days was significantly more active than when administered over 5 days, or as a single administration. To determine whether there was similar schedule-dependency for temozolomide, the antitumor activity of this agent was examined when given daily for 5 days, 2 x 5 days per 21 day cycle or 3 x 5 days per 28 day cycle. See, as incorporated by reference, Houghton, P.J. et al., Clinical Cancer Res. Vol. 6, 4110-4118, October 2000. The total dose per cycle was constant. Results for NB-1382 xenografts demonstrated the most pronounced schedule-dependent activity of temozolomide. In this experiment mice received a cumulative dose of 210 mg/kg per cycle. Mice received 3 cycles of treatment (total cumulative dose was 630 mg/kg). When temozolomide was given over 5 days (42 mg/kg/dose) CRs were achieved in all mice, and were maintained at week 12. Lower doses given over more protracted periods were progressively less effective. Far less schedule-dependent antitumor activity was observed in five other xenograft lines (data not shown), however, in all experiments administration of temozolomide in the most intensive schedule (daily for 5 consecutive days per cycle) was either more active or equally active compared to the other schedules. Consequently, for combination studies we selected the daily x 5 schedule for combination with CPT-11.

Dose levels of CPT-11 and temozolomide were chosen so that neither drug alone caused CR. For CPT-11 dose levels between 2.5 mg/kg and 0.18 mg/kg daily were administered, determined by the intrinsic sensitivity of any particular xenograft line. These dose levels give SN-38 systemic exposures consistent with those achieved in patients receiving CPT-11 using the same schedule of administration. Temozolomide was administered at dose levels ranging from 66 to 19 mg/kg, and are consistent with doses in patients giving achievable levels of parent drug and MTIC the active metabolite. The activity of combinations was significantly better than the activity of either single agent used at the same dose level, with a few exceptions. For example, in studies where monotherapy with either CPT-11 or temozolomide at the doses used caused maintained CR, it was not possible to determine whether combinations were superior to monotherapy (e.g. Rh30 and NB-1643). However, against several tumor lines temozolomide combined with CPT-11 demonstrated significant activity against tumors at dose levels that had little activity when administered as monotherapy. For example, against NB-SD neuroblastoma xenografts, temozolomide had little activity at tolerated dose levels, and similarly tumors progressed in mice treated with CPT-11 at 0.4 mg/kg. In combination these agents induced CR that was maintained at week 12. The glioblastoma line, SJ-GBM2, has a similar phenotype to NB-SD, except in this tumor MGMT is not detected. Although temozolomide induced some PRs, and an occasional CR during the 8 weeks of treatment at 66 or 42 mg/kg, the higher dose level resulted was lethal during cycle 3 of treatment. At 42 mg/kg the overall effect of treatment was stasis, as at the end of treatment tumor volumes were similar to that at initiation of temozolomide. CPT-11 combined with temozolomide (66 or 42 mg/kg) resulted in CR of all tumors with no tumor re-growth during the period of observation (12 weeks). Further, combination with CPT-11 decreased the toxicity of temozolomide (see later). NB-1771 tumors have detectable MGMT, and appear MMR proficient. These tumors were relatively sensitive to temozolomide as a single agent, hence for this study the dose was reduced to 19 mg/kg. CPT-11 induced some CRs at 1.25 mg/kg, and an occasional CR at 0.61 mg/kg, however at week 12 all tumors had progressed. Temozolomide combined with CPT-11 resulted in CRs of all tumors. In groups of mice receiving the higher dose of CPT-11 there was a single tumor that regrew, whereas at the lower dose 4 tumors regrew during the period of observation. Rh18 rhabdomyosarcoma expresses high MGMT levels and is deficient in MLH1 expression. Consequently, this xenograft is poorly sensitive to temozolomide as a single agent, lneffective doses of temozolomide combined with doses of CPT-11, which alone induced fewfRs, resulted in a high frequency of CRs.

To determine whether the antitumor activity observed with the drug combination coutd be a result of one agent altering the systemic exposure to the other, detailed pharmacokinetic studies were performed. Temozolomide and MTIC concentrations exceeded the limit of assay sensitivity for the duration of the study. Following administration, the apparent tₘₐₓ was 30 minutes for temozolomide and 1 hour for MTIC. The Cₘₐₓ for temozolomide and MTIC were 36 µg/mL and 0.8 µg/mL, respectively. The plasma AUC_{0→∞} for temozolomide and MTIC were 47 and 1.3 mg/L-hr, respectively. Irinotecan, SN-38, and APC concentrations exceeded the limit of assay sensitivity for the duration of the study. Following administration, the apparent tₘₐₓ was 30 minutes, 15 minutes, and 1 hour for irinotecan, SN-38, and APC, respectively. The Cₘₐₓ for irinotecan, SN-38, and APC were 1243, 84, and 41 µg/mL, respectively. The plasma AUC_{0→∞} for irinotecan, SN-38, and APC were 1821, 283, and 66 mg/L-hr, respectively..

In summary, combination of temozolomide and CPT-11 administered on optimal schedules is effective in inducing CR in a series of xenografts derived , from childhood solid malignancies. See also Patel V.J., et al., Clin. Canc. Res., Vol. 6, 4154-4157, October 2000.

### E. Clinical Study Design

The following Clinical Study Design may be used to treat cancer patients in accordance with the method of the present invention. Many modifications of this Clinical Study Design protocol will be apparent to the skilled clinician.

### Type of Study

This open-label, rising multiple-dose study is designed to characterize the safety profile and determine the maximum tolerated dose (MTD) and dose limiting toxicity (DLT) of oral temozolomide when administered in combination with irinotecan to adult patients with recurrent, persistent or progressive solid tumors.

### Treatment

Subjects will receive temozolomide once daily for 14 days (Day 1-14) followed by 14 days rest. The oral dosing will resume on Day 15 through day 21 followed by a 7 day-rest (Day 22-28). Irinotecan will be administered on Day 8 as a single IV bolus infusion. The cycle will be repeated every 28/29 days. At each does level, full evaluation of the patient will be performed. Evaluations will be performed on Day 1, 8, 15, and on Day 28/29.

All baseline evaluations will be performed within 14 days (inclusive) prior to administration of temozolomide. After reviewing all inclusion and exclusion criteria and obtaining written informed consent, patients will be assigned to the appropriate dose-level by the Principal Investigator. Cohorts of 3 patients each will be treated according to the dose levels noted in the Table 2 below. Once DLT is established, three additional patients will be treated at the next lower dose level (unless 6 patients have already been treated at that prior dose). This dose level will define the MTD (maximum tolerated dose).

There will be no intra-patient dose escalation. After the results of the Day 28/29 evaluation have been reviewed, patients may restart temozolomide on their assigned daily schedule until they develop severe Grade 3 or 4 non-hematologic or Grade 4 hematologic toxicity or disease progression.

All Grade 1 or greater hematologic or non-hematologic toxicity must have resolved prior to continuation of therapy. Delays of up to 2 weeks will be allowed for recovery, but if recovery has not occurred, the patient will be withdrawn from the study (see below; Duration of Study).

At each dose level, all patients must have completed one cycle of chemotherapy before starting the enrollment of subjects in the next cohort.

### Duration of Study

At each dose level, patients will receive once daily oral dosing of temozolomide on days 1-14 and a single IV infusion of irinotecan on Day 8. One cycle will last for 28 days. The cycle will be repeated until disease progression and/or DLT is recorded. The screening evaluations will be performed within 14 days (inclusive) prior to administration of temozolomide. Radiologic examinations within 30 days of initial dose administration will be used. After reviewing all inclusion and exclusion criteria, and after obtaining written informed consent, patients will be assigned to the appropriate dose level by the Principal Investigator. Cohorts of 3 patients each will be treated according to the dose levels noted in the Table 2 below. Dosing will be initiated at dose Level 1.

**Table 2: Dose Escalation/De-escalation Schedule**

| **Dose Level** | **CPT-11*** | **Temozolomide** |
|---|---|---|
| -3 | 100 mg/m² Day 8 | 75 mg/m²day Days 1-14 |
| -2 | 150 mg/m² Day 8 | 75 mg/m²day Days 1-14 |
| -1 | 175 mg/m² Day 8 | 100 mg/m²day Days 1-14 |
| 1 | 200 mg/m² Day 8 | 100 mg/m²day Days 1-14 |
| 2 | 200 mg/m² Day 8 | 125 mg/m²day Days 1-14 |
| 3 | 250 mg/m² Day 8 | 125 mg/m²day Days 1-14 |
| 4 | 250 mg/m² Day 8 | 150 mg/m²day Days 1-14 |
| 5 | 300 mg/m² Day 8 | 150 mg/m²day Days 1-14 |
| 6 | 350 mg/m² Day 8 | 150 mg/m²day Days 1-14 |

| | | |
|---|---|---|
| *Administration: IV infusion over 30 minutes | | |

There will be no within patient dose escalation. After the results of the Days 27-29 evaluation have been reviewed, patients may be restarted on their assigned schedule until they develop severe (Grade 3 or 4) or serious toxicity or disease progression.

Dose-limiting toxicity (DLT) will be defined from the safety profile during the first 28-day cycle for each dose level. If no dose-limiting toxicity is seen in the cohort of patients at a given dose level, new patients may be treated at the next level. When drug-induced DLT is encountered during the first cycle of therapy in 1 patient, a maximum of 6 patients may be treated at that level. If DLT is not observed in the additional patients, new patients may be treated at the next higher dose level. When a minimum of 2 patients experience DLT at a given level, this dose level will be the DLT dose level. However, additional patients may experience DLT due to the timing of patient enrollment onto that dose level. A maximum of 6 patients may be treated at the DLT dose level.

Once DLT is established, subsequent patients should be treated at the next lower dose level. Six patients must be treated at the MTD. The MTD is the dose level at which 0/6 or 1/6 patients experience DLT with at least two patients experiencing DLT at the next higher dose level.

Patients will have met the minimum safety study requirements if they have been treated for one cycle of treatment or experience DLT. Patients who discontinue from the study before meeting these study requirements will be regarded as unevaluable for safety evaluation and will be replaced.

Following completion of review of Cycle 1 Days 28-29 labs, patients may receive subsequent treatment with temozolomide and irinotecan based upon the absence of unacceptable toxicity and/or progression of disease. Patients may continue treatment at the same dose level upon satisfactory resolution of any adverse effects, not defined as dose limiting. If unacceptable toxicity defined as dose-limiting toxicity occurs, drug is to be withdrawn. Upon resolution of the toxicity, patients may continue treatment at one dose level below the dose level administered. If more than two-dose level reductions are required for continued treatment of any patient, then the patient must be withdrawn from the study. Subjects who experience dose-limiting toxicity in dose Level-1 will not be eligible to receive additional dosing. If 2 or more patients experience Grade 3-4 adverse events at dose level 1, a de-escalation schedule (see Table 2 above) will be used for the next cohort of 3 patients which will be enrolled at dose level-1. The NCI-Common Toxicity Criteria (CTC) Version 2.0 will be used to assess the adverse events.

### Patient inclusion criteria:

- Adult patients (≥ 18 year old) with histologically proven advanced solid tumors refractory to conventional therapy or chemo-naïve patients with advanced cancers.
- If prior treatment, at least:
   - 4 weeks since major surgery
   - 1 week since minor surgery
   - 4 weeks since prior RT or chemotherapy or until previous chemotherapy related toxicity has abated, except for ≥ 6 weeks for nitrosoureas, L-PAM, mitomycin C, strontium 89 or samarium
   - 3 weeks since prior therapy with biological agents (e.g. bi-specific antibodies, IL-2, interferon)
   Patients must have no known hypersensitivity to temozolomide or irinotecan.
- Performance score ≤ 2 (ECOG scale).
- Patients must be able to give written informed consent.
- No evidence of other malignancies/treatment at other sites within 5-years with the exception of carcinoma in-situ of the cervix and adequately treated basal or squamous cell carcinoma of the skin.

### Patient exclusion criteria:

- Evidence of renal impairment as based on a serum creatinine ≥ 2X the upper limit of normal.
- Patients who received high dose chemotherapy and stem cell transplant.
- Patients heavily pretreated (4 or more chemotherapy regimens).
- Patients with life threatening conditions or severe pre-existing condition (e.g. severe CAD, CHF, severe COPD, etc).
- Evidence of significant bone marrow suppression with baseline ANC ≤1,500/µl and/or platelet count of ≤100.000/µL
- SGOT or SGPT greater than or equal to 3 times upper limit of normal (5 fold if elevations are due to liver metastases).
- Patients with known bone marrow involvement with tumor as assessed by the Principal Investigator.
- Patients who are poor medical risks because of non-malignant systemic disease as well as those with active uncontrolled infection.
- Frequent vomiting or medical condition, which could interfere with oral medication intake (e.g., partial bowel obstruction, partial intestinal bypass, and external biliary diversion).
- Wide Field Radiation therapy (≥25% of bone marrow) within four weeks prior to administration of temozolomide/irinotecan (pelvic radiation is considered 25% of bone marrow reserve.
- Patients who have received investigational therapy of any type within 4 weeks prior to administration of temozolomide/irinotecan.
- Lack of resolution of all toxic manifestations of prior chemotherapy to Grade 1, biologic or radiation therapy (alopecia excluded).
- Prior allogeneic, syngeneic or autologous bone marrow transplantation or stem cell transplantation.
- Known HIV positivity or AIDS-related illness.
- Pregnant or nursing women.
- Men or Women of childbearing potential who are not using an effective method of contraception. Women of childbearing potential must have a negative urine pregnancy test 24 hours prior to first administration of temozolomide/irinotecan.

### Replacement of Subjects

Subjects who withdraw from the study prior to Days 27-29 of the first cycle of therapy other than for serious adverse events or dose limiting toxicity will be defined as dropouts and will be replaced. Replacement subjects will be allocated the next subject number.

### TREATMENTS

Patients will be sequentially enrolled into the following dose cohorts as described in Table 2 above.

Enrollment into the subsequent dose level will not occur until all three subjects have completed 1 cycle of therapy. There will be no within patient.dose escalation. During treatment periods, the appropriate dose of temozolomide will be administered before bedtime. Capsules of temozolomide are available in 20, 100 and 250-mg strengths. All doses will be rounded up to the nearest 20-mg to accommodate capsule strength. The exact dose administered will be recorded. Each dose of temozolomide should be given with the least number of capsules. Temozolomide should be given with approximately 8 ounces of water over a short time. Patients should be instructed to swallow capsules whole and in rapid succession and to not chew the capsules. If vomiting occurs during the course of treatment, no re-dosing of the patient is allowed before the next scheduled dose. Dose limiting toxicity is defined during the first treatment cycle. If DLT occurs in the first cycle, the subject should be withdrawn from the study. If DLT occurs beyond cycle one then the subject can have the dose adjusted to the next lower level. If dose-limiting toxicity is encountered at a dose level in one patient during the first cycle of therapy, at least 3 additional patients may be treated at that level (for a total of 6 patients).

If DLT is not observed in the additional patients, new patients may be treated at the next level. When a minimum of 2 patients experience DLT at a given level, patient entry at that dose level is stopped, the MTD has been exceeded and dose escalation will be stopped. However, additional patients may experience DLT due to the timing of patient enrollment. A maximum of 6 patients may be treated at DLT level.

Once DLT is established, three additional patients will be treated at the next lower dose level (unless six patients have already been treated at that prior dose). If two or more patients experience DLT at the lower dose level, then the MTD has again been exceeded and dose escalation will be stopped and three additional patients will be treated at the next lower dose (unless six patients have already been treated at that dose level). The MTD is the dose level at which 0/6 or 1/6 patients experience DLT during their first two treatment cycles with at least two patients encountering DLT at the next higher dose level.

### Treatment Criteria for Subsequent Treatment Beyond First Course

If the following criteria are not met, treatment should be held for 1 week and the patient should be reevaluated weekly until the following laboratory values are met:
1. Platelets ≥100,000/µL
2. ANC ≥1500/µL
3. Hemoglobin ≥8.5 mg/dL

### Treatment Criteria for Subsequent Cycles Beyond Second Course

In the absence of disease progression or unacceptable toxicity, patients may continue to receive treatment with temozolomide and irinotecan until the occurrence of disease progression or unacceptable toxicity according to the guidelines stated below.

### Criteria for Subsequent Treatment

The initiation of the treatment cycles with irinotecan and temozolomide, 28 days after the first daily dose of temozolomide, will be based upon complete blood counts (CBC) obtained within 72 hours prior to starting the next treatment (Day 1). Growth factors cannot be used to induce elevations in neutrophil or platelet count for the purposes of administration of irinotecan and temozolomide on the scheduled dosing interval.

If temozolomide cannot be administered on the scheduled day of dosing, because of delayed recovery from toxicity the CBC will be repeated weekly for up to and including 2 weeks until the ANC is ≥1,500/mm³ and platelet count ≥100,000/mm³. If ANC remains <1,500/mm³ or platelet count <100,000/ mm³ at 2 weeks or patient is required more than two dose level reductions, the Principal Investigator will consider withdrawing the subject from the study. All non-hematologic Grade 2,3 and 4 toxicities must have resolved to at least to Grade 1 or baseline level defined by the inclusion criteria prior to repeat dosing.

Patients who experience Grade 3 non-hematological or Grade 4 hematological toxicity but who recover within 2 weeks will be treated for subsequent cycles at the next lower dose level (e.g. from dose level 2 to dose level 1, etc).

Patients who experience severe and/or or prolonged diarrhea (Grade 3) at Dose Level 1 will have the dose of irinotecan reduced from 200 mg/m²to 175 mg/m² in the subsequent cycles. Early-onset diarrhea and/or delayed-diarrhea will be treated as per irinotecan (Camptosar® ) product insert.

### Non-Study Treatments

### Concomitant Medications

All medications administered within 4 weeks prior to administration of temozolomide and all concomitant therapy administration during the study with the reasons for therapy use will be documented. Any chemotherapy, biologic and radiation therapy administered prior to registration onto the study will be documented. Prior surgery will also be documented.
The patient may receive prophylactic antiemetics for all doses of temozolomide and irinotecan at the discretion of the treating physician.

Other chemotherapy, radiation, or biologic therapy may not be used while the patient is on study. Colony stimulating factors including erythropoietin may not be used to prevent the occurrence of myelotoxicity, but may be used for therapy of severe or serious bone marrow suppression.

All patients should be maintained on the same medications throughout the study period, as medically feasible.

### Safety and Tolerance

The primary objectives are to evaluate the safety and tolerability of temozolomide in combination with irinotecan (Camptosar® ) and to define dose limiting toxicities (if any) and the maximum tolerated dose (if one is observed). Safety will be assessed based on physical examination, vital signs and clinical laboratory test results. Tolerability will be assessed based on signs and subjective symptoms of adverse events.

### Definition of Dose-Limiting Toxicity

1. ANC <500/µL for longer than 5 days.
2. ANC <500/µL with fever.
3. Platelets <10,000/µL (Grade 4).
4. Any Grade 3 non-hematologic toxicity or Grades 3-4 nausea/vomiting while receiving an optimal antiemetic regimen for prophylaxis and management (i.e., consisting of a 5HT₃ antagonist on an optimal dose-schedule).
5. Any Grade 4 diarrhea while receiving optimal anti-diarrhea medication.
6. Treatment delays of greater than 2 weeks for toxicity.

### Statistical Considerations

The primary objective of the study is to determine the dose-limiting toxicity (DLT) and maximum tolerated dose (MTD) of temozolomide in combination with irinotecan in patients with advanced cancer. MTD is defined as the dose level where the true toxicity rate is no higher than 30% and will be based on the toxicity profile over the first cycle. This study is designed to enroll 3 patients at most dose levels, with a maximum of 6 patients at DLT level and a total of 6 patients at the MTD level. The MTD is estimated as the dose level where 0 or 1 out of 6 patients experience DLT with at least 2 patients experiencing DLT at the next higher level (which will be the DLT dose level). If the true toxicity rate at a dose level is 'P' then the probability of declaring the dose level as toxic (DLT level) is as follows per Table 3:

**Table 3**

| Toxicity Rate (P) | Probability of DLT |
|---|---|
| 0.2 | 0.345 |
| 0.3 | 0.580 |
| 0.4 | 0.767 |
| 0.5 | 0.891 |
| 0.6 | 0.959 |
| 0.7 | 0.989 |
| 0.8 | 0.998 |

With the design and the sample size used for this study, doses with high toxicity rates (≥ 40%) have a high probability of (>0.70) being declared as DLT.

### Tumor Response

Patients will be clinically evaluated for tumor response after each 28-day course of treatment. Patients with tumor lesions requiring radiographic studies (CT, MRI, ultrasound, X-ray) will have these studies at Screening, and repeated for those continuing treatment every two months. The same method, either similar radiographic study or physical examination, throughout the study must measure all lesions so that the comparison is consistent. Criteria required for determining partial or complete response should be present for at least 4 weeks. Tumor response will be determined radiographically at the time of withdrawal from the study if not performed within the previous 21 days.

### Definition of Measurability of Disease (Modified from WHO Reporting of Response)

### Evaluable Disease but Non-Measurable

a) Lesions in previously irradiated fields, except CNS lesions;
b) Ascites and pleural effusions
c) Lymphangitic pulmonary metasases
d) Abdominal masses that can be palpated but not measured

### Bidimensionally Measurable Disease ("Measurable lesions") Examples of such lesions include:

a) A lung tumor surrounded by aerated lung
b) A skin nodule
c) A superficial lymph node

### Non-measurable Disease, Lesions considered to be truly non-measurable include the following:

a) Bone lesions
b) Leptomeningeal disease
c) Ascites
d) Pleural/pericardial effusion
e) Inflammatory breast disease
f) Lymphangitis cutis/pulmonis
g) Abdominal masses that are not confirmed and followed by imaging techniques
h) Cystic Lesions

### CT, MRI Scan, Ultrasonography, Chest X-Ray

CT scan, MRI scan or Ultrasonography may be used to measure malignant lesions provided that at least one lesion has a diameter greater or equal to 2 cm.

### Criteria for Tumor Response (Modified from WHO Reporting of Response)

Ideally, all measurable lesions should be measured at each assessment. When multiple lesions are present in any organ, this may not be possible and, under such circumstances, up to 6 representative lesions should be selected, if available.

### Measurable Disease:

**Complete Response (CR)** Complete disappearance of all clinically detectable malignant disease, determined by 2 observations not less than 4 weeks apart.

**Partial Response (PR)** In case of bidimensionally measurable disease, decrease by at least 50% of the sum of the products of the largest perpendicular diameters of all measurable lesions as determined by 2 observations not less than 4 weeks apart. For unidimensionally measurable disease, decrease by at least 50% in the sum of the largest diameters of all lesions as determined by 2 observations not less than 4 weeks apart. It is not necessary for all lesions to have regressed to qualify for partial response, but no lesion should have progressed and no new lesion should appear. Serial evidence of appreciable change documented by copies of radiographic studies must be obtained and must be available for subsequent review. The assessment must be objective.

**Stable Disease (SD)** For bidimensionally measurable disease, <50% decrease or <25% increase in the sum of the products of the largest perpendicular diameters of all measurable lesions. For unidimensionally measurable disease, <50% decrease or <25% increase in the sum of the diameters of all lesions. No new lesions should appear.

**Progressive Disease (PD)** 25 % increase in the size of at least one bidimensionally (product of the largest perpendicular diameters) or unidimensionally measurable lesion or appearance of a new lesion. The occurrence of pleural effusion or ascites is also considered as progressive disease if this is substantiated by positive cytology. Pathological fracture or collapse of bone is not necessarily evidence of disease progression.

**Overall Patient Tumor Response** Determination of overall patient tumor response for uni-and bidimensionally measurable disease will be done according to Table 4 below:

**Table 4**

| Response in Bidimensionally Measurable Disease | Response in Unidimensionally Measurable Disease | Overal Patient Tumor Response |
|---|---|---|
| PD | Any | PD |
| Any | PD | PD |
| SD | SD or PR | SD |
| SD | CR | PR |
| PR | SD or PR or CR | PR |
| CR | SD or PR | PR |
| CR | CR | CR |

| | | |
|---|---|---|
| PD: Progressive Disease | | |
| CR: Complete Response | | |
| PR: Partial Response | | |
| SD: Stable Disease | | |

### Evaluation of Response in the Presence of Non-Measurable Disease

Non-measurable disease will not be used in the assessment of overall patient tumor response except in the following situations:
a) Overall complete response: if non-measurable disease is present, it should disappear completely. Otherwise, the patient cannot be considered as an "overall complete responder."
b) Overall progression: in case of a significant increase in the size of non-measurable disease or the appearance of a new lesion, the overall response will be progression.

The present invention is useful for the treatment of cancer. The precise dosage and dosage regimen may be varied by the attending clinician in view of the teachings herein depending upon the requirements of the patient, e.g., the patient's age, sex and the severity of the cancer being treated. Determination of the proper dosage and dosage regimen for a particular patient will be within the skill of the attending clinician.

## Claims

1. The use of a therapeutically effective amount of temozolomide for the preparation of a pharmaceutical composition for treating a human patient afflicted with cancer, wherein the treatment comprises administering temozolomide and irinotecan to such a patient for a time period sufficient to effect at least a partial tumor response.

2. The use of a therapeutically effective amount of irinotecan for the preparation of a pharmaceutical composition for treating a human patient afflicted with cancer, wherein the treatment comprises administering temozolomide and irinotecan to such a patient for a time period sufficient to effect at least a partial tumor response.

3. The use of claim 1 or 2, wherein the irinotecan is in the form of a hydrochloride salt.

4. The use of claim 1 or 2, wherein the temozolomide and irinotecan are administered over repeated 21 day cycles, where said 21 day cycles are divided into three 1 week periods.

5. The use of claim 4, wherein the total amount of irinotecan administered over the 21 day period ranges from 3 to 400 mg/m2 of the patient's body surface.

6. The use of claim 4, wherein the amount of temozolomide administered over the 21 day period ranges from 50-200 mg/m²/day of the patient's body surface and wherein said temozolomide is administered for 5-14 days over the 21 day period.

7. The use of claim 4, wherein the amount of temozolomide administered over the 21 day period ranges from 50-200 mg/m²/day of the patient's body surface and wherein said temozolomide is administered for 5-14 days over the 21 day period and the irinotecan is in the form of a hydrochloride salt.

8. The use of claim 4, wherein the irinotecan is administered for 5 consecutive days at a daily dose of 10 to 40 mg/m²/day during the first week and for 5 consecutive days at a daily dose of 10 to 40 mg/m²/day during the second week, followed by the third week in which irinotecan is not administered.

9. The use of claim 8, wherein the temozolomide is administered for 5 consecutive days at a daily dose of 100 to 200 mg/m²/day during the first week, followed by the second and third week in which temozolomide is not administered.

10. The use of claim 8, wherein the temozolomide is administered for 5 to 7 consecutive days at a daily dose of 100 to 200 mg/m²/day during the first and third weeks of the 21 day cycle.

11. The use of claim 8, wherein the temozolomide is administered for 5 to 7 consecutive days at a daily dose of 100 to 200 mg/m²/day during the first and second weeks of the 21 day cycle.

12. The use of claim 10 or 11, wherein the irinotecan is in the form of a hydrochloride salt.

13. The use of claim 4, wherein the irinotecan is administered on a single day of the 21 day cycle in an amount of from 250 to 650 mg/m².

14. The use of claim 4, wherein the irinotecan is administered once a week during the 21 day cycle at a dose of from 100 to 125 mg/m².

15. The use of claim 4, wherein the temozolomide and irinotecan are both administered on the first day of the 21 day treatment cycle.

16. The use of claim 15, wherein the irinotecan is in the form of a hydrochloride salt.

17. The use of claim 1 or 2, wherein the temozolomide is administered prior to the administration of the irinotecan.

18. The use of claim 4, wherein the temozolomide and irinotecan are administered in three 21 day cycles, each cycle having a dosing period wherein the temozolomide is administered for the first five days of the 21 day cycle at a daily dose of 50 to 200 mg/m²/day, the irinotecan is administered with the temozolomide for the first 5 days of the 21 day cycle and for an additional 5 day period during the second week of the 21 day cycle at a daily dose of 10 to 40 mg/m²/day, followed by the third week in which temozolomide and irinotecan is not administered.

19. The use of claim 17, wherein the temozolomide is administered orally and the irinotecan is administered intravenously.

20. The use of claim 1 or 2, wherein the temozolomide and irinotecan are administered over repeated 28 day cycles.

21. The use of claim 20, wherein the total amount of irinotecan administered over the 28 day period ranges from 3 to 400 mg/m² of the patient's body surface.

22. The use of claim 20, wherein the amount of temozolomide administered over the 28 day period ranges from 50-200 mg/m²/day of the patient's body surface and wherein said temozolomide is administered for 5-14 days over the 28 day period.

23. The use of claim 20, wherein the amount of temozolomide administered over the 28 day period ranges from 50-200 mg/m²/day of the patient's body surface and wherein said temozolomide is administered for 5-14 days over the 28 day period and the irinotecan is in the form of a hydrochloride salt.

24. The use of claim 20, wherein temozolomide and irinotecan are administered over a 28 day cycle, wherein the temozolomide is administered on days 1-14 of said cycle at a daily dose of 75 to 150 mg/m²/day and wherein the irinotecan is administered on day 8 of said cycle at a daily dose of 100 to 350 mg/m²/day.

25. The use of claim 24, wherein the temozolomide is administered orally and the irinotecan is administered intravenously.

26. A medical kit for treating a cancer patient is provided, comprising :
(a) a supply of temozolomide ;
(b) a supply of irinotecan ; and
(c) printed instructions for administering temozolomide and irinotecan to a cancer patient.

## Patentansprüche

1. Verwendung einer therapeutisch wirksamen Menge von Temozolomid zur Herstellung einer pharmazeutischen Zusammensetzung zur Behandlung eines Menschen, der unter Krebs leidet, wobei die Behandlung die Verabreichung von Temozolomid und Irinotecan an einen entsprechenden Patienten für eine ausreichende Zeit umfasst, um mindestens eine partielle Tumorreaktion zu bewirken.

2. Verwendung einer therapeutisch wirksamen Menge von Irinotecan zur Herstellung einer pharmazeutischen Zusammensetzung zur Behandlung eines Menschen, der unter Krebs leidet, wobei die Behandlung die Verabreichung von Temozolomid und Irinotecan an einen entsprechenden Patienten für eine ausreichende Zeit umfasst, um mindestens eine partielle Tumorreaktion zu bewirken.

3. Verwendung gemäß Anspruch 1 oder 2, bei der das Irinotecan in Form des Hydrochloridsalzes vorliegt.

4. Verwendung gemäß Anspruch 1 oder 2, bei der das Temozolomid und das Irinotecan über sich wiederholende Zyklen von 21 Tagen verabreicht werden, wobei die Zyklen von 21 Tagen in drei einwöchige Phasen aufgeteilt werden.

5. Verwendung gemäß Anspruch 4, bei der die Gesamtmenge an Irinotecan, die über die Phase von 21 Tagen verabreicht wird, im Bereich von 3 bis 400 mg/m² der Körperoberfläche des Patienten beträgt.

6. Verwendung gemäß Anspruch 4, bei der die Menge an Temozolomid, die über die Phase von 21 Tagen verabreicht wird, 50-200 mg/m²/Tag der Körperoberfläche des Patienten beträgt und bei der das Temozolomid für 5-14 Tage der 21-tägigen Phase verabreicht wird.

7. Verwendung gemäß Anspruch 4, bei der die Menge an Temozolomid, die über die Phase von 21 Tagen verabreicht wird, 50-200 mg/m²/Tag der Körperoberfläche des Patienten beträgt und bei der das Temozolomid für 5-14 Tage der 21-tägigen Phase und das Irinotecan in Form eines Hydrochloridsalzes verabreicht wird.

8. Verwendung gemäß Anspruch 4, bei der das Irinotecan für fünf aufeinander folgende Tage in einer täglichen Dosierung von 10 bis 40 mg/m²/Tag während der ersten Woche und für fünf aufeinander folgende Tage in einer täglichen Dosierung von 10 bis 40 mg/m²/Tag über die zweite Woche verabreicht wird, gefolgt von einer dritten Woche, in der Irinotecan nicht verabreicht wird.

9. Verwendung gemäß Anspruch 8, bei der das Temozolomid für fünf aufeinander folgende Tage in einer täglichen Dosierung von 100 bis 200 mg/m²/Tag während der ersten Woche verabreicht wird, gefolgt von einer zweiten und dritten Woche, in der Temozolomid nicht verabreicht wird.

10. Verwendung gemäß Anspruch 8, bei der das Temozolomid für 5 bis 7 aufeinander folgende Tagen in einer täglichen Dosierung von 100 bis 200 mg/m²/Tag während der ersten und zweiten Woche des 21-tägigen Zyklus verabreicht wird.

11. Verwendung gemäß Anspruch 8, bei der das Temozolomid für 5 bis 7 aufeinander folgende Tagen in einer Dosierung von 100 bis 200 mg/m²/Tag während der ersten und zweiten Woche des 21-tägigen Zyklus verabreicht wird.

12. Verwendung gemäß Anspruch 10 oder 11, bei der das Irinotecan in Form eines Hydrochloridsalzes vorliegt.

13. Verwendung gemäß Anspruch 4, bei der das Irinotecan an einem einzigen Tag des 21-tägigen Zyklus in einer Menge von 250 bis 650 mg/m² verabreicht wird.

14. Verwendung gemäß Anspruch 4, bei der das Irinotecan während des 21-tägigen Zyklus einmal pro Woche in einer Dosierung von 100 bis 125 mg/m² verabreicht wird.

15. Verwendung gemäß Anspruch 4, bei der das Temozolomid und das Irinotecan beide am ersten Tag des 21-tägigen Behandlungszyklus verabreicht werden.

16. Verwendung gemäß Anspruch 15, bei der das Irinotecan in Form eines Hydrochloridsalzes verabreicht wird.

17. Verwendung gemäß Anspruch 1 oder 2, bei der das Temozolomid vor der Verabreichung des Irinotecan gegeben wird.

18. Verwendung gemäß Anspruch 4, bei der das Temozolomid und das Irinotecan in drei 21-tägigen Zyklen verabreicht werden, jeder Zyklus eine Dosierungsphase aufweist, in der das Temozolomid die ersten 5 Tage des 21-tägigen Zyklus in einer Dosierung von 50 bis 200 mg/m²/Tag verabreicht wird, das Irinotecan mit dem Temozolomid für die ersten 5 Tage des 21-tägigen Zyklus und für eine weitere Phase von 5 Tagen während der zweiten Woche des 21-tägigen Zyklus in einer Dosierung von 10 bis 40 mg/m²/Tag verabreicht wird, gefolgt von einer dritten Woche, in der Temozolomid und Irinotecan nicht verabreicht werden.

19. Verwendung gemäß Anspruch 17, bei der das Temozolomid oral und das Irinotecan intravenös verabreicht werden.

20. Verwendung gemäß Anspruch 1 oder 2, bei der Temozolomid und Irinotecan über sich wiederholende Zyklen von 28 Tagen verabreicht werden.

21. Verwendung gemäß Anspruch 20, bei der die Gesamtmenge an Irinotecan, die über die Phase von 28 Tagen verabreicht wird im Bereich von 3 bis 400 mg/m² der Körperoberfläche des Patienten beträgt.

22. Verwendung gemäß Anspruch 20, bei der die Menge an Temozolomid, die über die Phase von 28 Tagen verabreicht wird, von 50-200 mg/m²/Tag der Körperoberfläche des Patienten beträgt und bei der das Temozolomid für 5-14 Tage der 28-tägigen Phase verabreicht wird.

23. Verwendung gemäß Anspruch 20, bei der die Menge an Temozolomid, die über die Phase von 28 Tagen verabreicht wird, von 50-200 mg/m²/Tag der Körperoberfläche des Patienten beträgt und bei der das Temozolomid für 5-14 Tage der 28-tägigen Phase und das Irinotecan in Form eines Hydrochloridsalzes verabreicht wird.

24. Verwendung gemäß Anspruch 20, bei der Temozolomid und Irinotecan über einen 28-tägigen Zyklus verabreicht werden, wobei das Temozolomid an Tagen 1-14 des Zyklus in einer Dosierung von 25-150 mg/m²/Tag und das Irinotecan an Tag 8 des Zyklus in einer Dosierung von 100 bis 350 mg/m²/Tag verabreicht wird.

25. Verwendung gemäß Anspruch 24, bei der Temozolomid oral und das Irinotecan intravenös verabreicht werden.

26. Medizinisches Kit zur Behandlung von Krebspatienten, umfassend:
(a) einen Vorrat an Temozolomid;
(b) einen Vorrat an Irinotecan; und
(c) gedruckte Anweisungen für die Verabreichung von Temozolomid und Irinotecan an einen Krebspatienten.

## Revendications

1. Utilisation d'une quantité thérapeutiquement efficace de témozolomide pour la préparation d'une composition pharmaceutique destinée à traiter un patient humain atteint d'un cancer, le traitement consistant à administrer du témozolomide et de l'irinotécan à un tel patient pendant une période de temps suffisante pour présenter au moins une réponse tumorale partielle.

2. Utilisation d'une quantité d'irinotécan thérapeutiquement efficace pour la préparation d'une composition pharmaceutique destinée à traiter un patient humain atteint d'un cancer, le traitement consistant à administrer du témozolomide et de l'irinotécan à un tel patient pendant une période de temps suffisante pour présenter au moins une réponse tumorale partielle.

3. Utilisation conforme à la revendication 1 ou la revendication 2, dans laquelle l'irinotécan est sous la forme d'un sel chlorhydrate.

4. Utilisation conforme à la revendication 1 ou la revendication 2, dans laquelle le témozolomide et l'irinotécan sont administrés au cours de cycles répétés de 21 jours, lesdits cycles de 21 jours étant divisés en trois périodes d'une semaine.

5. Utilisation conforme à la revendication 4, dans laquelle la quantité totale d'irinotécan administrée au cours de la période de 21 jours est comprise entre 3 et 400 mg/m² de la surface corporelle du patient.

6. Utilisation conforme à la revendication 4, dans laquelle la quantité de témozolomide administrée au cours de la période de 21 jours est comprise entre 50 et 200 mg/m²/jour de la surface corporelle du patient et dans laquelle ledit témozolomide est administré pendant 5 à 14 jours au cours de la période de 21 jours.

7. Utilisation conforme à la revendication 4, dans laquelle la quantité de témozolomide administrée au cours de la période de 21 jours est comprise entre 50 et 200 mg/m²/jour de la surface corporelle du patient et dans laquelle ledit témozolomide est administré pendant 5 à 14 jours au cours de la période de 21 jours et l'irinotécan est sous la forme d'un sel chlorhydrate.

8. Utilisation conforme à la revendication 4, dans laquelle l'irinotécan est administré pendant 5 jours consécutifs en une dose quotidienne de 10 à 40 mg/m²/jour pendant la première semaine et pendant 5 jours consécutifs en une dose quotidienne de 10 à 40 mg/m²/jour pendant la deuxième semaine, puis la l'irinotécan n'est pas administré la troisième semaine.

9. Utilisation conforme à la revendication 8, dans laquelle le témozolomide est administré pendant 5 jours consécutifs en une dose quotidienne de 100 à 200 mg/m²/jour pendant la première semaine, puis le témozolomide n'est pas administré la deuxième et la troisième semaines.

10. Utilisation conforme à la revendication 8, dans laquelle le témozolomide est administré pendant 5 à 7 jours consécutifs en une dose quotidienne de 100 à 200 mg/m²/jour pendant la première et la troisième semaines du cycle de 21 jours.

11. Utilisation conforme à la revendication 8, dans laquelle le témozolomide est administré pendant 5 à 7 jours consécutifs en une dose quotidienne de 100 à 200 mg/m²/jour pendant la première et la deuxième semaines du cycle de 21 jours.

12. Utilisation conforme à la revendication 10 ou la revendication 11, dans laquelle l'irinotécan est sous la forme d'un sel chlorhydrate.

13. Utilisation conforme à la revendication 4, dans laquelle l'irinotécan est administré un seul jour du cycle de 21 jours en une quantité comprise entre 250 et 650 mg/m².

14. Utilisation conforme à la revendication 4, dans laquelle l'irinotécan est administré une fois par semaine pendant le cycle de 21 jours en une dose comprise entre 100 et 125 mg/m².

15. Utilisation conforme à la revendication 4, dans laquelle le témozolomide et l'irinotécan sont tous deux administrés le premier jour du cycle de traitement de 21 jours.

16. Utilisation conforme à la revendication 15, dans laquelle l'irinotécan est sous la forme d'un sel chlorhydrate.

17. Utilisation conforme à la revendication 1 ou la revendication 2, dans laquelle le témozolomide est administré avant l'administration de l'irinotécan.

18. Utilisation conforme à la revendication 4, dans laquelle le témozolomide et l'irinotécan sont administrés en trois cycles de 21 jours, chaque cycle ayant une période de dosage où le témozolomide est administré pendant les cinq premiers jours du cycle de 21 jours en une dose quotidienne de 50 à 200 mg/m²/jour, l'irinotécan est administré avec le témozolomide pendant les 5 premiers jours du cycle de 21 jours et pendant une période supplémentaire de 5 jours pendant là deuxième semaine du cycle de 21 jours en une dose quotidienne de 10 à 40 mg/m²/jour, puis le témozolomide et l'irinotécan ne sont pas administrés la troisième semaine.

19. Utilisation conforme à la revendication 17, dans laquelle le témozolomide est administré par voie orale et l'irinotécan est administré par voie intraveineuse.

20. Utilisation conforme à la revendication 1 ou la revendication 2, dans laquelle le témozolomide et l'irinotécan sont administrés au cours de cycles répétés de 28 jours.

21. Utilisation conforme à la revendication 20, dans laquelle la quantité totale d'irinotécan administrée au cours de la période de 28 jours est comprise entre 3 et 400 mg/m² de la surface corporelle du patient.

22. Utilisation conforme à la revendication 20, dans laquelle la quantité de témozolomide administrée au cours de la période de 28 jours est comprise entre 50 et 200 mg/m²/jour de la surface corporelle du patient et dans laquelle ledit témozolomide est administré pendant 5 à 14 jours au cours de la période de 28 jours.

23. Utilisation conforme à la revendication 20, dans laquelle la quantité de témozolomide administrée au cours de la période de 28 jours est comprise entre 50 et 200 mg/m²/jour de la surface corporelle du patient et dans laquelle ledit témozolomide est administré pendant 5 à 14 jours au cours de la période de 28 jours et l'irinotécan est sous la forme d'un sel chlorhydrate.

24. Utilisation conforme à la revendication 20, dans laquelle le témozolomide et l'irinotécan sont administrés au cours d'un cycle de 28 jours, le témozolomide étant administré les jours 1 à 14 dudit cycle en une dose quotidienne de 75 à 150 mg/m²/jour et l'irinotécan étant administré le jour 8 dudit cycle en une dose quotidienne de 100 à 350 mg/m²/jour.

25. Utilisation conforme à la revendication 24, dans laquelle le témozolomide est administré par voie orale et l'irinotécan est administrée par voie intraveineuse.

26. Kit médical destiné à traiter un patient atteint d'un cancer, comprenant :
(a) une alimentation en témozolomide ;
(b) une alimentation en irinotécan ;
(c) et des instructions imprimées pour administrer le témozolomide et l'irinotécan à un patient atteint d'un cancer.
